# EUROPEAN PATENT SPECIFICATION

(11) **EP 1 827 342 B1**
(45) Date of publication and mention of the grant of the patent: **24.08.2011**
(21) Application number: 05819794.8
(22) Date of filing: 19.12.2005
(51) Int. Cl.: A61F 15/00

(54) **LIQUID-RESISTANT PROTECTION FOR AN EXTREMITY**
FLÜSSIGKEITSRESISTENTER SCHUTZ FÜR EINE EXTREMITÄT
PROTECTION RESISTANTE AUX LIQUIDES POUR UNE EXTREMITE

(30) Priority: 20.12.2004 SE 0403097; 20.12.2004 US 636871 P
(43) Date of publication of application: 05.09.2007
(73) Proprietor: Andersson, Tommy, 605 58 Norrköping (SE); Sandell, Jan-Eric, 605 95 Norrköping (SE)
(72) Inventor: Andersson, Tommy, 605 58 Norrköping (SE); Sandell, Jan-Eric, 605 95 Norrköping (SE)
(74) Representative: Bokinge, Ole
(86) International application number: PCT/SE2005/001981
(87) International publication number: WO 2006/068601

(56) References cited:
- EP-A- 0 230 775
- EP-A- 0 937 447
- WO-A1-94/24971
- DE-U1- 9 205 982
- US-A- 4 523 586
- US-A- 5 083 557
- US-A- 5 152 282
- US-A- 5 342 286
- US-A- 5 643 183

## Description

### Background of the invention

Using a liquid-resistant protection for extremities, such as an arm or a leg, in order to protect open wounds, applied plaster of Paris or adhesive plasters, for example, on these parts of the body from wet and moisture when showering and/or bathing, for example, is already known.

EP-0 695 157 discloses such a liquid-resistant protection comprising a plastic sleeve with one open end and one end which has been sealed by heat sealing. In addition the liquid-resistant protection comprises a sealing strip which over its entire length comprises a tape, which is provided with an adhesive surface and a protective film covering this surface. The strip is furthermore of the same length as the sleeve and is fastened to the sleeve by way of a weld.

A further known liquid-resistant protection, which comprises a sock-shaped sleeve of a plastic film having an opening and an edge surrounding the opening, is described in US 4, 727, 864. The protection further comprises a sealing strip of elastic material having an adhesive surface over its length At one end the strip is fastened by ties on the outside and the edge of the sleeve. On the inside of the sleeve adjoining the edge there is also a smaller section of adhesive surface for fastening a part of the edge directly to the skin.

DE 92 05 982 U1 discloses a wound dressing in the shape of a tube with two open ends. At both ends, the dressing is provided with Velcro® fastening means which comprises a fastening strip comprising a first part of the Velcro fastening means, which is fastened to a second part of the Velcro® fastening means that is provided on a strip, which in turn is attached to the wound dressing tube. The term Velcro® means fastening means consisting of a strip of nylon with a surface of minute hooks that may be fasten to a corresponding strip with a surface of uncut pile. Such fastening means is meant for repeated use. The document discloses two separate sets of Velcro® fastening means, which both are fastened to the wound dressing tube.

EP A 0 230 775 discloses a protection device for fitment around a limb to prevent water contact with e.g. a dressing. The protection device comprises a sleeve portion and sealing portion. According to one embodiment the sealing portion may be in the form of a strap, which is heat sealed to the sleeve. The strap may be coated with an adhesive on both faces and may be secured to the sleeve by an intermediate member. The intermediate portion may be of a gauze-backed Sellotape adhesive material. The strap may be passes around the limb to be protected and above the sleeve and then secured to the intermediate member. The intermediate member adheres readily to both the sleeve and the strap.

US 0 230 775 discloses a podiatry boot provided with fastening means comprising an anchoring web and a fastening web. The fastening web is made up of strip of adhesive with an inner and an outer surface and is permanently attached to an flexible sheet. A part of the inner surface of the fastening web is permanently attached to the anchoring web by an adhesive coating and the other part may be separated from the anchoring web. By the wearer wrapping the leg portion of the boot around his leg a loose flap is formed, which may be secured to the boot by pressing the intermediate adhesive portion of the fastening web onto the flap. The fastening web hence makes up a strip which at one end is detachable from a release coating on the flexible sheet and which strip may be used to secure a flap formed by the wearer.

US A 5 342 286 discloses a waterproof protection for extremities comprising a sack-like member, with a front and rear panel defining the covering. The rear panel is provided with a lip extending above the opening of the covering. The lip is provided with a strip of adhesive, which covers the entire width of the lip from one side to the other, the adhesive strip is covered with a removable protection. The lip has a lateral perforation extending approximately four-ffths of the width thereof. In use the perforation is torn and the lip hence becomes a strip (tie member) which at the non-perforated end still is attached to the rear panel (securing portion). The securing portion is adhered to the wearers skin and the tie member is wrapped around the extremity to secure the covering to the extremity

EP A 0 937 447 discloses protective clothing for medical use. The clothing is provided with sealing means comprising elastic annular sealing strips and/or inflatable elastic tubular elements, a "border". The strips may be treated or impregnated with a soil, non-irritating surface means facing the inside for a close adherence to the skin during sealing. The sealing may be further improved by the inflatable elements, which are held in place by a non elastic belt.

One problem of known liquid-resistant protections is that they are difficult to handle when fastening the liquid-resistant protection. A further problem is that they do not stay tight, especially when bathing, that is to say when the extremity is fully immersed in water.

### Disclosure of the invention

An object of the present invention is to provide an improved liquid-resistant protection, which entirely or partially eliminates the problems with the prior art.

The object is fully or partially achieved by a liquid-resistant protection and a set of parts according to the respective independent claim.

Embodiments of the invention are set forth in the dependent claims.

According to an aspect, a liquid-resistant protection for an extremity is therefore provided comprising a sleeve of a waterproof polymer film, the sleeve having an open end. An adhesive layer for sealing the open end of the sleeve is arranged on a section of the outside of the sleeve at the end of the sleeve, close to the open end. Furthermore, the adhesive layer is a part of the outer surface of the sleeve. The open end of the sleeve is tightenable around an extremity introduced into the sleeve so that excess material of the sleeve forms a flap. This flap is bendable towards the outside of the sleeve, such that the flap adheres to the outside of the sleeve by means of the adhesive layer wherein a part of one side of the flap comprises a part of or the entire section having the adhesive layer. In this way a tight fold of the sleeve can be provided for providing a liquid-resistant protection around the extremity.

Such an improved liquid-resistant protection provides an improved liquid-resistant protection which is easy to apply and which stays tight.

According to out embodiment the liquid-resistant protection may comprise two open ends. This embodiment is suitable for the liquid resistant protection of an inner part of an extremity, such as an upper arm, for example.

According to one embodiment the liquid-resistant protection may comprise an open end and a closed end. This embodiment is suitable for the liquid-resistant protection of an outer part of art extremity, such as a hand or foot, for example.

According to one embodiment the liquid-resistant protection may further comprise an elongate sealing strip, one surface of which has an adhesive layer. Such a sealing strip affords the advantage that the sealing of the sleeve around an extremity can be improved. This allows an extremity with an injury, for example, to be fully immersed in water.

According to one embodiment the adhesive layer on the outside of the sleeve may consist of a double-sided adhesive tape. One advantage of such an adhesive layer is that it is easy to manufacture and apply to the sleeve.

According to on embodiment the section having the adhesive layer on the outside of the sleeve may run over half of the circumference of the sleeve. This affords the advantage that a simple and liquid-tight fastening of the sleeve is obtained when applying it to an extremity. In addition the adhesive layer is easy to apply to the sleeve during manufacture.

According to one embodiment the liquid-resistant protection may further comprise a venting valve for removing air included in the liquid-resistant protection. Such a venting valve means that an extremity, which has been provided with a liquid-resistant protection enclosing an injury, for example, can be fully immersed in water, without the water pressure causing the included air in the sleeve to destroy the liquid-tight sealing of the sleeve.

Such a set of parts provides an improved liquid-resistant protection compared to known liquid-resistant protections, giving a liquld-resistant protection which remains tight, especially when bathing, when the extremity is fully immersed in water.

### Brief description of the drawings

Fig. 1 is a schematic perspective view showing a liquid-resistant protection,
Fig. 2 is a schematic perspective view showing a liquid-resistant protection,
Fig. 3 is a schematic perspective view showing a liquid-resistant protection,
Fig. 4a-4b illustrates an example of how a liquid-resistant protection can be applied to a lower arm, Fig. 4a is an embodiment of the invention,
Fig. 5a-5b illustrates an example of how a liquid-resistant protection according to Fig. 2 or 3 can be applied to a lower arm,
Fig. 6 is a schematic perspective view showing a liquid-resistant protection comprising a venting valve.

### Description of embodiments

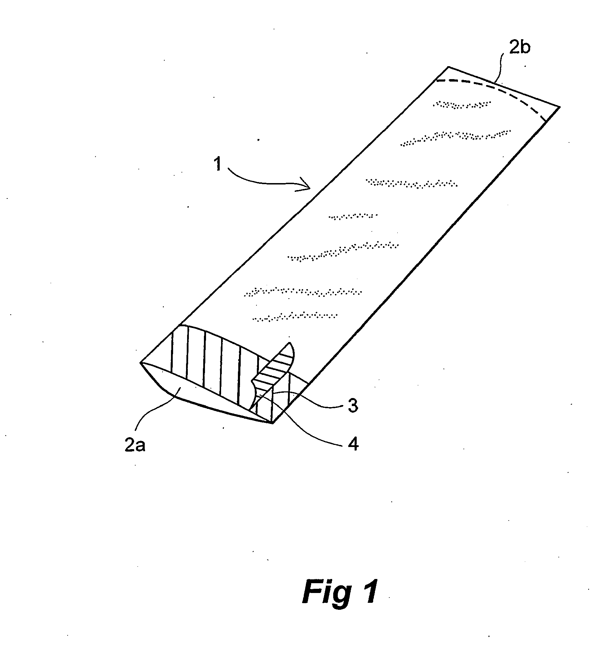

As is shown in Figs. 1 to 3, a liquid-resistant protection may be formed by sealing one end of a plastic sleeve in order to form a bag 1 having an open end 2a and a closed end 2b. Unless otherwise specified, the sleeve 1 will consequently be referred to below as a bag 1.
Fig. 1 shows a liquid-resistant protection, which comprises an elongate, sleeve-like bag 1 of a waterproof polymer film. The polymer film may be composed of thin olefin plastic, such as ethylene plastics, propylene plastics or the like, or compounds thereof. The polymer film may further comprise additives such as ethylene butyl acrylate (EBA) in order to make the polymer film softer, more flexible and tougher. Other polymer materials and additives known to the person skilled in the art, which are suitable for forming a waterproof polymer film and which can be used in contact with the skin, may also be used. The bag 1 has an open end 2a through which an extremity, such as a part of an arm, a leg or a foot can be inserted. An adhesive layer 3 is arranged on a section 3 on the outside of the bag adjoining the open end. In the embodiment shown the section 3 with the adhesive layer 3 runs over basically half of the circumference of the bag. The adhesive layer 3 may also be covered with a protective film 4 maintaining the liquid-resistant protection. This protective film 4 may be designed so that it can easily be separated from the adhesive layer 3.

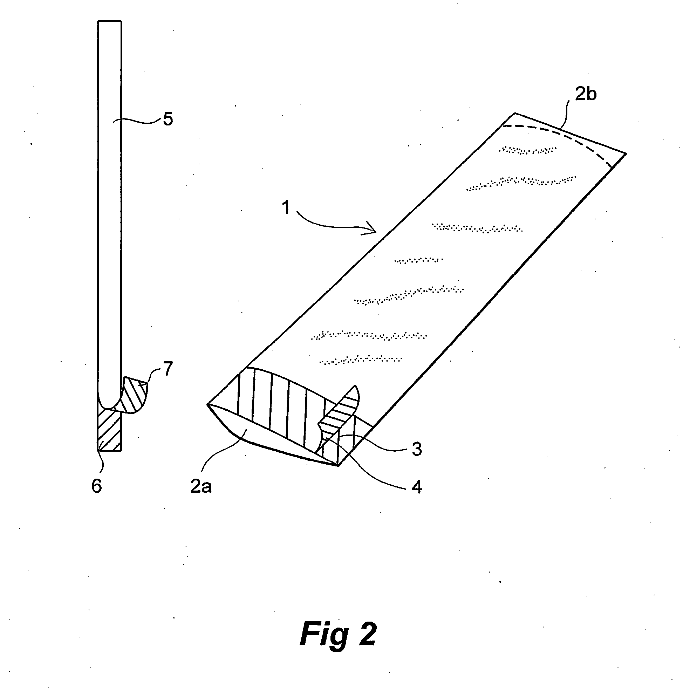

Fig. 2 shows a liquid-resistant protection, which comprises a bag 1 similar to the embodiment of the bag 1 shown in Fig. 1. The protection according to Fig. 2 further comprises an elongate sealing strip 5, which like the bag may be composed of an olefin plastic. One surface 6 of the sealing strip 5 may have an adhesive layer 6. The adhesive layer 6 may also be covered with a protective film 7 maintaining the liquid-resistant protection. This protective film 7 can easily be separated from the adhesive layer 6. The sealing strip 5 may be integrated with the bag 1 or separate and packed in a set of parts together with the bag 1.

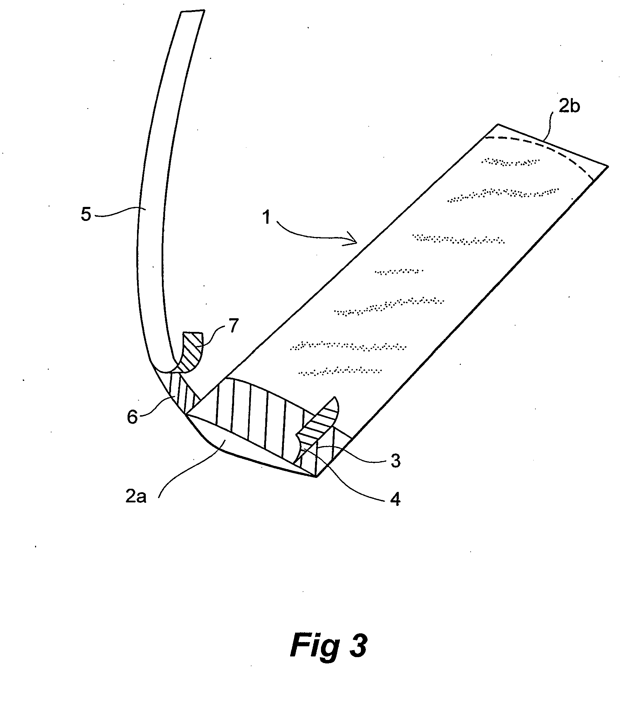

Fig. 3 shows a variant of the bag 1 shown in Fig. 2. The bag 1 shown in Fig. 3 differs from that shown in Fig. 2 in that one end of the sealing strip 5 is fastened to the bag 1 adjoining the open end 2a. The sealing strip 5 may be fastened to the bag 1 by adhesive means, for example, or by means of a weld. Other ways of fastening such a sealing strip 5 known to the person skilled in the art may also be used.

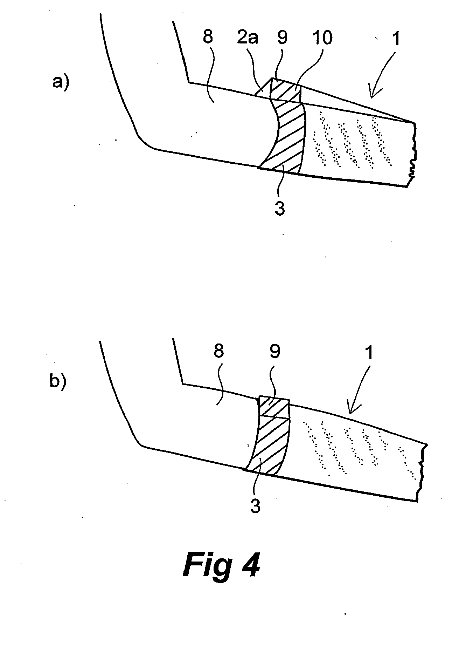

Fig. 4a-4b illustrate how the bag 1 shown in Fig. 1 is applied to a lower arm 8. As can be seen from Fig. 4a, a lower arm 8 has been inserted in through the open end 2a. The open end 2a of the bag 1 is then tightened around the lower arm 8, so that excess material forms a flap 9. A part of one side 10 of the flap comprises a part of or the entire section having the adhesive layer 3. The protective film 4 is then removed and the flap 9 is bent over so that the adhesive layer 3 is fastened to the part of the outside of the bag adjoining the open end 2a and resting flat against the skin (Fig. 4b). This method provides an easy way of fitting the liquid-resistant protection and at the same time a good protection against moisture, especially if contact is achieved between two sections of the adhesive layer, counteracting the formation of a duct through the fold.

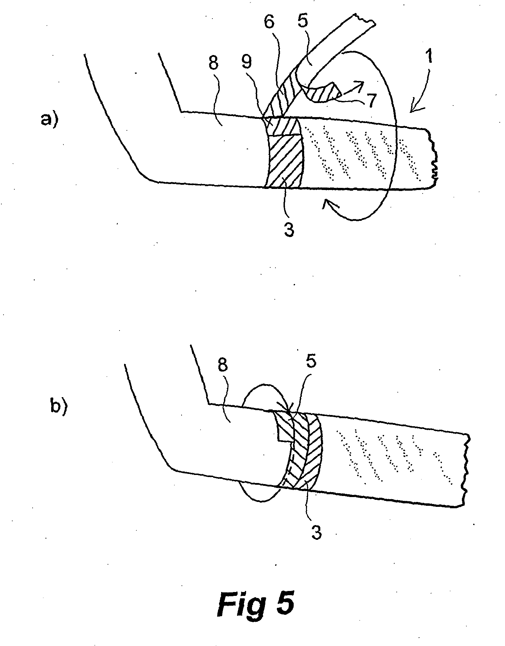

Fig. 5a-5b illustrates how a sealing strip 5 can be applied in order to further improve the protection against moisture. The bag 1 is first applied as described above in connection with Fig. 4a-4b. The protective film 7 is then separated from the sealing strip 5, which is in turn fastened to the outside of the bag 1 already applied in Fig. 4b, adjoining the open end 2a. The sealing strip 5 is then wrapped around the arm 8 so that it finally covers an area comprising a part of the section of the bag 1 at the open end 2a, which encloses the extremity and the skin bordering this section.

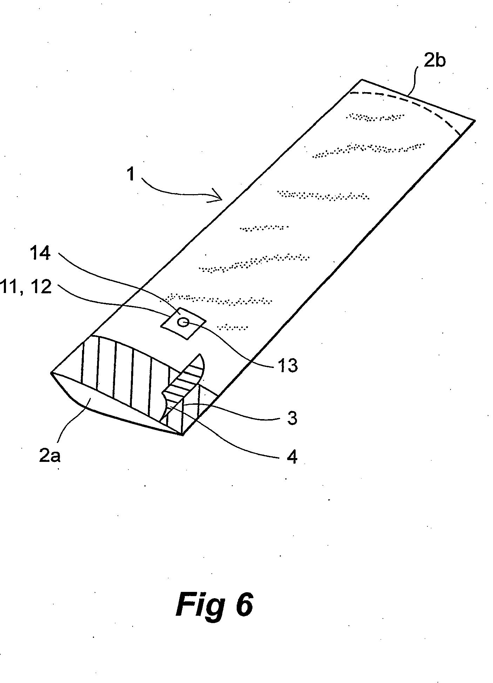

As shown in Fig. 6, the liquid-resistant protection may further comprise a venting valve 11. This venting valve 11 allows an extremity, which has been provided with a liquid-resistant protection enclosing an injury, for example, to be fully immersed in water. Without this venting valve 11 the water pressure would force included air inside the bag 1 out through the sealed open end 2a of the bag, which would result in destruction of the tight seal at the open end 2a of the bag 1. The venting valve 11 is suitably arranged on a section of the bag 1 adjoining the open end 2a of the bag 1, since the extremity provided with liquid-resistant protection is suitably immersed with that part of the extremity enclosed by the closed end 2b of the bag 1 being immersed first, followed by the part of the extremity enclosed by the open end 2a. The venting valve 11 may be fitted to the bag 1 before or after applying the liquid-resistant protection to an extremity as described above. One example of a venting valve 11 is a non-return valve 11, which allows included air in the bag 1 to flow out and which prevents liquid getting into the inside of the bag 1. The construction of such a non-return valve 11 will be familiar to the person skilled in the art and may comprise a valve body 12 having a through duct 13 in which, for example, a diaphragm, such as a rubber diaphragm, is designed to produce the function of the non-return valve. The valve body 12 may furthermore comprise a plate 14 through which the duct 13 runs in a direction at right angles to the plane of the plate 14. This plate 14 allows the valve to be fitted to the bag 1. For example, an adhesive layer may be arranged on one surface of the plate 14 for fitting to the bag 1. The venting valve 11 may furthermore be designed with means of penetrating the polymer film of the bag 1 and producing an opening over which the venting valve 11 can be fitted (not shown). In the absence of such means, the opening may be achieved by other means, such as a pen.

According to one embodiment the venting valve 11 may comprise an open duct 13 without a non-return valve function. In this embodiment the duct 13 may be sealed, for example, by means of a tape once the air has been forced out and before the section with the venting valve 11 is immersed in water.

According to one embodiment the adhesive layer 3 on the bag 1 may comprise a double-sided adhesive tape. It will be appreciated that other types of adhesive layer 4 may also be used, such as a layer of adhesive applied to the bag 1. It will be further appreciated that in various embodiments the section having the adhesive layer 3 may cover a greater or lesser part of the circumference and the length of the bag 1. The adhesive layer 3 must comprise of a part of the outer surface of the bag large enough to form a tight fold.

According to one embodiment the shape of the bag 1 is optional and may be adjusted to the contour of the extremity that is to be protected from liquid. An example of an optional shape of the bag 1 is a sock-shaped bag, which is intended for the liquid-resistant protection of a foot.

According to one embodiment the sealing strip 5 is of a length at least equal to the circumference of the extremity around which the sealing strip 5 is to be wound. This length permits a secure sealing of the bag 1 around the extremity, which provides a liquid-resistant protection which remains tight, especially when bathing, when the extremity is fully immersed in water.

A liquid-resistant protection is furthermore provided comprising a sleeve 1 of a waterproof polymer film, the sleeve 1 having two open ends 2a. An adhesive layer 3 for sealing the sleeve 1 is arranged in a section 3 on the outside of the sleeve 1 adjoining each of the open ends 2a. It will be appreciated that this embodiment may also comprise two sealing strips 5. Such a sleeve 1 having two open ends may be suitable for the liquid-resistant protection of the inner part of an extremity, such as an upper arm. The embodiment comprising a bag 1 having one open end 2a and one closed end 2b may be suitable for the liquid-resistant protection of an outer part of an extremity, such as a hand or foot. The sleeve 1 can thus be adapted to cover only the necessary part of an extremity.

## Claims

1. Liquid-resistant protection for an extremity comprising a sleeve (1) of a waterproof polymer film, the sleeve (1) having an open end (2a), wherein an adhesive layer (3) for sealing the open end (2a) of the sleeve (1) is arranged on the sleeve (1),
**characterised in that**
the adhesive layer (3) is a part of the outer surface of the sleeve (1) on a section (3) of the sleeve (1) at the end of the sleeve (1), close to the open end (2a),
wherein the open end (2a) of the sleeve is tightenable around an extremity (8) introduced into the sleeve (1) so that excess material of the sleeve (1) forms a flap (9),
wherein the flap (9) is bendable towards the outside of the sleeve (1), such that the flap (9) adheres to the outside of the sleeve (1) by means of the adhesive layer (3) wherein a part of one side (10) of the flap (9) comprises a part of or the entire section (3) having the adhesive layer (3),
wherein a tight fold of the sleeve (1) can be provided for providing a liquid-resistant protection around the extremity.

2. Liquide-resistant protection according to Claim 1, comprising two open ends (2a).

3. Liquide-resistant protection according to Claim 1, comprising one open end (2a) and one closed end (2b).

4. Liquide-resistant protection according to Claim 1, further comprising an elongats sealing strip (5), one surface of which has an adhesive layer (6).

5. Liquide-resistant protection according to Claim 1, wherein the adhesive layer (3) is a double-side adhesive tape.

6. Liquid-resistant protection according to Claim 1, wherein the section (3) having the adhesive layer (3) runs over half the circumference of the sleeve (1).

7. Liquide-resistant protection according to Claim 1, further comprising a venting valve (11) for removing air included in the liquide-resistant protection.

## Patentansprüche

1. Flüssigkeitsbeständiger Schutz für eine Extremität, umfassend eine Hülle (1) aus einer wasserfesten Polymerfolie, wobei die Hülle (1) ein offenes Ende (2a) aufweist, wobei eine Klebstoffschicht (3) zum Versiegeln des offenen Endes (2a) der Hülle (1) auf der Hülle (1) angeordnet ist,
**dadurch gekennzeichnet, dass**
die Klebstoffschicht (3) ein Teil der äußeren Oberfläche der Hülle (1) auf einem Abschnitt (3) der Hülle (1) an dem Ende der Hülle (1) ist, welches nahe am offenen Ende (2a) liegt,
wobei das offene Ende (2a) der Hülle um eine in die Hülle (1) eingeführte Extremität (8) abdichtbar ist, so dass überschüssiges Material der Hülle (1) eine Falte (9) bildet,
wobei die Falte (9) gegen die Außenseite der Hülle (1) abbiegbar ist, so dass die Falte (9) an der Außenseite der Hülle (1) mittels der Klebstoffschicht (3) anhaftet, wobei ein Teil der einen Seite (10) der Falte (9) einen Teil von oder den gesamten Abschnitt (3) umfasst, welcher die Klebstoffschicht (3) aufweist,
wobei ein dichter Falz der Hülle (1) bereitgestellt werden kann, um einen flüssigkeitsbeständigen Schutz um die Extremität zu gewährleisten.

2. Flüssigkeitsbeständiger Schutz gemäß Anspruch 1, umfassend zwei offene Enden (2a).

3. Flüssigkeitsbeständiger Schutz gemäß Anspruch 1, umfassend ein offenes Ende (2a) und ein geschlossenes Ende (2b).

4. Flüssigkeitsbeständiger Schutz gemäß Anspruch 1, ferner umfassend einen länglichen Dichtstreifen (5), dessen eine Seite eine Klebstoffschicht (6) aufweist.

5. Flüssigkeitsbeständiger Schutz gemäß Anspruch 1, wobei die Klebstoffschicht (3) ein doppelseitiges Klebeband ist.

6. Flüssigkeitsbeständiger Schutz gemäß Anspruch 1, wobei der Abschnitt (3) mit der Klebstoffschicht (3) sich über die Hälfte des Umfangs der Hülle (1) erstreckt.

7. Flüssigkeitsbeständiger Schutz nach Anspruch 1, ferner umfassend ein Lüftventil (11) zum Entfernen von Luft, die in dem flüssigkeitsbeständigen Schutz eingeschlossen ist.

## Revendications

1. Protection résistante aux liquides pour une extrémité comprenant un manchon (1) d'un film polymère étanche à l'eau, le manchon (1) ayant une extrémité ouverte (2a), où une couche adhésive (3) permettant de sceller l'extrémité ouverte (2a) du manchon (1) est agencée sur le manchon (1),
**caractérisée en ce que**
la couche adhésive (3) constitue une partie de la surface externe du manchon (1) sur une section (3) du manchon (1) à l'extrémité du manchon (1), proche de l'extrémité ouverte (2a),
où l'extrémité ouverte (2a) du manchon peut être serrée autour d'une extrémité (8) introduite dans le manchon (1) de sorte que le matériau en excès du manchon (1) forme un battant (9),
où le battant (9) peut être fléchi vers l'extérieur du manchon (1), de telle sorte que le battant (9) adhère à l'extérieur du manchon (1) au moyen de la couche adhésive (3), où une partie d'un côté (10) du battant (9) comprend une partie ou l'intégralité de la section (3) comportant la couche adhésive (3) ;
où un pli serré du manchon (1) peut être prévu pour conférer une protection résistante aux liquides autour de l'extrémité.

2. Protection résistante aux liquides selon la revendication 1, comprenant deux extrémités ouvertes (2a).

3. Protection résistante aux liquides selon la revendication 1, comprenant une extrémité ouverte (2a) et une extrémité fermée (2b).

4. Protection résistante aux liquides selon la revendication 1, comprenant en outre une bande de scellage allongée (5), dont une surface comporte une couche adhésive (6).

5. Protection résistante aux liquides selon la revendication 1, dans laquelle la couche adhésive (3) est une bande adhésive double face.

6. Protection résistante aux liquides selon la revendication 1, dans laquelle la section (3) comportant la couche adhésive (3) court sur la moitié de la circonférence du manchon (1).

7. Protection résistante aux liquides selon la revendication 1, comprenant en outre une soupape d'aération (11) permettant d'éliminer l'air inclus dans la protection résistante aux liquides.
